# EUROPÄISCHE PATENTSCHRIFT

(11) **EP 2 943 797 B1**
(45) Veröffentlichungstag und Bekanntmachung des Hinweises auf die Patenterteilung: **17.10.2018**
(21) Anmeldenummer: 14790648.1
(22) Anmeldetag: 30.10.2014
(51) Int. Cl.: G01N 35/02, B01L 3/00, B01L 9/00

(54) **MIKROPLATTENREADER MIT INKUBATIONSVORRICHTUNG**
MICROPLATE READER WITH INCUBATION DEVICE
LECTEUR DE MICROPLAQUES AVEC DISPOSITIF D'INCUBATION

(30) Priorität: 07.11.2013 CH 18672013
(43) Veröffentlichungstag der Anmeldung: 18.11.2015
(73) Patentinhaber: Tecan Trading AG, 8708 Männedorf (CH)
(72) Erfinder: WENCZEL, Gyoergy, A-5201 Seekirchen (AT); GEBETSROITHER, Harald, A-5082 Grödig (AT); MENGES, Friedrich, 83471 Berchtesgaden (DE)
(74) Vertreter: OK pat AG
(86) Internationale Anmeldenummer: PCT/EP2014/073389
(87) Internationale Veröffentlichungsnummer: WO 2015/067529

(56) Entgegenhaltungen:
- EP-A1- 1 623 759
- WO-A1-02/24336
- WO-A1-96/21855
- WO-A1-03/089137
- US-A- 6 097 025

## Beschreibung

Die Erfindung betrifft einen Mikroplattenreader mit Inkubationsvorrichtung zum Reduzieren der Flüssigkeits-Verdunstung aus Wells von Mikroplatten.

Mikroplatten-Reader, mit denen auf optischem Wege die Inhalte von einem oder mehreren Wells einer Mikroplatte untersucht bzw. analysiert werden können, sind seit langem bekannt. Als Mikroplatte werden im Zusammenhang mit der vorliegenden Erfindung alle Multiwellplatten bezeichnet, die eine Vielzahl an Wells oder Behältern aufweisen, welche in einem Array angeordnet sind. Speziell bevorzugte Mikroplatten weisen zumindest annähernd die Masse und die Standfläche einer Mikroplatte nach dem SBS Standard auf, wie dieser vom American National Standards Institute (ANSI) veröffentlicht wurde. Bekannt sind beispielsweise solche Standard-Mikroplatten, deren Wells mit einem Rundboden, Flachboden oder V-Boden ausgestattet sind. Allen diesen Standard-Mikroplatten mit den unterschiedlichsten Wellformen ist gemeinsam, dass der Achsabstand der jeweils in einem Array angeordneten Wells ebenfalls normiert ist (vgl. ANSI_SBS 1-2-3-4-2004 Standard für Mikroplatten-Dimensionen aus dem Jahr 2006). Dieser Achsabstand beträgt z.B. bei 24-Well (4 x 6) Platten 18 mm, bei 96-Well (8 x 12) Platten 9 mm, bei 384-Well (16 x 24) Platten 4.5 mm und bei 1536-Well (32 x 48) Platten 2.25 mm. Die Höhe einer Standard-Mikroplatte kann je nach Typ sehr stark variieren und beträgt typischerweise zwischen 10.4 mm (z.B. 1536 V-Boden Deep Well Platte) und 44 mm (z.B. 96 Well Masterblock® von Greiner).

Bekannte Mikroplatten-Reader sind mit den entsprechenden Lichtquellen und/oder Detektoren ausgerüstet, um Proben in den Wells von Mikroplatten anhand ihrer Absorption, und/oder Fluoreszenz und/oder Lumineszenz zu untersuchen. Üblicherweise befinden sich die Proben in einer Flüssigkeit, die den Einflüssen der Umwelt ausgesetzt ist. Insbesondere bei lange dauernden Versuchsreihen, die typischerweise in für sich stehenden Mikroplatten-Readern während Stunden oder gar Tagen und womöglich noch bei gegenüber der Raumtemperatur erhöhten Temperaturen durchgeführt werden, können sich für die Proben enthaltende Flüssigkeit Verdunstungsprobleme einstellen. Das Verdunsten der Probenflüssigkeit führt zu einer Eindickung und damit zu einer Änderung der Konzentration von Puffersubstanzen und zu untersuchenden Molekülen (Analyten). Dadurch werden beispielsweise die Wachstumsbedingungen für Zellbasierte Versuche oder die Reaktion von Zellen auf versuchsbedingte Einflüsse verändert. Es wurde zudem beobachtet, dass die Probenflüssigkeit von in den Ecken einer Standard-Mikroplatte angeordneten Wells deutlich mehr derartigen Verdunstungsproblemen ausgesetzt ist als von in der Mitte einer Mikroplatte angeordneten Wells. Dies wiederum bedeutet, dass die Eindickung nicht homogen über alle Wells einer Mikroplatte verteilt eintritt sondern zu Unterschieden, und damit zu nicht vergleichbaren Ergebnissen, innerhalb der gleichen Versuchsreihe führt.

Ein Mikroplatten-Reader ist unter anderem aus dem Patent US 6,097,025 bekannt. Eine in das Gerät eingesetzte Mikroplatte kann mittels einer Transportplatte gegenüber optischen Einrichtungen positioniert werden, wobei Messungen von oben ("top optic") oder von unten ("bottom optic") möglich sind.

Vorrichtungen zum Verhindern oder zumindest zum Reduzieren derartiger Verdunstungsprobleme sind aus dem Stand der Technik bekannt. So offenbart das Patent US 5,789,251 eine selbstklebende, mit einer Pipettenspitze durchstechbare Abdeckfolie zum Verschliessen der Wells einer Standard-Mikroplatte; allerdings kann eine derartige Abdeckfolie das optische Auswerten der Versuchsergebnisse im Mikroplatten-Reader beeinträchtigen, so dass diese Folie hochtransparent ausgebildet sein muss und keine Kondensationsprodukte an deren Innenoberfläche aufweisen darf. Andernfalls muss die Folie für die optische Auswertung entfernt werden, was schlimmstenfalls zu einem Verschütten oder sonstigen Verändern der Proben (z.B. durch Kreuzkontamination) führen kann. Aus dem Patent US 6,408,595 B1 ist ein recht aufwändiger Applikator bekannt, mit dem selbstklebende Abdeckfolien auf Standard-Mikroplatten aufgebracht werden können.

Aus dem Patent US 6,518,059 B1 ist bekannt, eine Standard-Mikroplatte in einer Klimakammer mit eingebautem Flüssigkeitsreservoir aufzubewahren und so das Auftreten von Verdunstungsproblemen zu verhindern; allerdings muss die Mikroplatte nach der Inkubationszeit aus der Klimakammer genommen und für die optische Auswertung zu einem Mikroplatten-Reader transferiert werden. Bei diesem Transfer und auch während der Zeit im Mikroplatten-Reader können ebenfalls Verdunstungsprobleme auftreten. US 2006/0023299 A1 offenbart ebenfalls eine Klimakammer, die innerhalb einer abdeckbaren, gegen magnetische Felder abschirmenden Inkubatorbox angeordnet ist. An Stelle der Klimakammer kann eine Mikroplatte in die mit doppelt umlaufenden Wänden ausgerüstete Inkubatorbox gelegt werden. Zwischen den beiden Wänden der Inkubatorbox ist ein Bad mit sterilisiertem Wasser angeordnet, mit welchem in der Inkubatorbox eine nahezu 100 %-ige Sättigung des Kulturgases mit Wasser erzielt werden kann. Dagegen offenbart die Patentanmeldung US 2008/0180793 A1 ein System mit einer in ein Lebendzell-Kammer eingesetzten Mikroplatte, deren Wells über ein Gasleitungssystem mit feuchtem Kulturgas versorgt werden, das in einer speziellen Aufbereitungsanlage des Systems mit Wasserdampf angereichert wird.

Auch das Verwenden von Abdeckvorrichtungen für Mikroplatten ist zum Verhindern von Verdunstungsproblemen bekannt. Das Patent EP 0 311 440 B1 offenbart eine Vorrichtung zum Durchführen von Flüssigkeitsreaktionen, bei welcher eine Mikroplatte zwischen zwei Heizplatten eingeklemmt wird. Dabei können Temperaturen zwischen 37 °C und 90 °C eingestellt werden, wobei die Wells der Mikroplatte mit einer elastischen Platte der oberen Heizplatte abgedeckt sind um das Verdunsten der Flüssigkeit in den Wells zu reduzieren. Das Patent EP 1 623 759 B1 offenbart ein System mit einer speziell konstruierten Mikroplatten/Deckel-Kombination, wobei die Umfassungswände der Mikroplatte und des Deckels so ausgebildet sind, dass sie ineinander greifen und so den Raum über den Wells abdichten. Die Verwendung von Standard-Mikroplatten ist bei diesem System nicht möglich.

Einen etwas anderen Weg offenbart das Patent US 7,767,154 B2, bei dem ein Deckel mit Rastzapfen so auf eine Mikroplatte gepresst wird, dass diese Rastzapfen mit Reibschluss in entsprechenden Rastöffnungen der mikroplatte gehalten werden. Eine umlaufende Dichtung am Deckel verringert zudem die Möglichkeit, dass aus den Wells stammende Dämpfe in die Umwelt entweichen können. Allerdings ist eine spezielle Ausgestaltung der Mikroplatte und des Deckels notwendig und das Verschliessen und Öffnen der Mikroplatten/Deckel-Kombination muss mit einem speziellen und recht komplizierten Werkzeug erfolgen. Die Verwendung von Standard-Mikroplatten ist hier ebenfalls nicht möglich.

Noch ein anderer Weg ist aus EP 1 465 730 B1 bekannt. Dort wird ein Deckel so auf die Wells einer Multiwellplatte gelegt, dass der Deckel die Probenflüssigkeit vollflächig berührt, wobei aus überfüllten Wells austretende Probenflüssigkeit als Dichtung verwendet wird. Dadurch besteht die Gefahr, dass zu analysierende Moleküle verloren gehen. Die Verwendung von Standard-Mikroplatten ist auch bei diesem System nicht möglich.

Das Dokument WO 02/24336 A1 offenbart eine Inkubationsvorrichtung mit einem Deckel und einem Halterahmen, in den eine oder mehrere Titerplatten eingelegt werden können. Der Deckel wird auf Dichtungen und den Halterahmen gepresst und ein Stempel verschliesst eine durchgehende Aussparung des Halterahmens unterhalb der Titerplatte, sodass die Titerplatte immer dicht eingeschlossen ist.

Das Dokument WO 03/089137 A1 offenbart ein System, eine Substratplatte (Mikroplatte) und eine Inkubationsvorrichtung zum Durchführen von biologischen Versuchen. Jeder Wellboden der Mikroplatte umfasst eine Mikroarraysubstrat mit gerichteten Durchflusskanälen und die Inkubationsvorrichtung umfasst eine Inkubationskammer zum Halten der Mikroplatte in einem Heizblock und einen Deckel zum Abdichten der Inkubationskammer. Der Deckel umfasst eine Dichtung, deren Öffnungen so in einem Array angeordnet sind, dass beim aufgelegtem Deckel jedes Well der Mikroplatte individuell abgedichtet wird.

Im Stand der Technik bestehende Nachteile umfassen die Tatsache, dass ein Injektor zum Zugeben von Reagenzien nicht eingesetzt werden kann, ohne dass die Folie von der Mikroplatte entfernt oder der Deckel vor dem Einlegend der Mikroplatte in einen Mikroplatten-Reader abgehoben werden muss (eine eventuelle Ausnahme stellt US 5,789,251 dar). Je kompletter die Abdichtung der Wells gegen die Umwelt ausgebildet ist, desto geringer ist der mögliche (und z.B. für Zellkulturen wichtige) Gasaustausch bei Zellbasierten Versuchen. Lose abgedeckte Mikroplatten erleiden trotzdem eine, möglicherweise verringerte, aber trotzdem bestehende Eindickung der Probenflüssigkeit durch Verdunstung. Kondensationsprodukte, die sich an der Unterseite von optisch transparenten Abdeckfolien oder Mikroplattendeckeln niederschlagen führen zu Falschmessungen im Absorbance-Modus. Abdeckfolien oder Deckel auf Mikroplatten verhindern Fluoreszenzmessungen im Top Detektions-Modus; Lumineszenzmessungen durch Mikroplattendeckel hindurch sind nicht möglich.

Es ist deshalb eine Aufgabe der vorliegenden Erfindung, einen Mikroplattenreader vorzuschlagen, mit dem aus dem Stand der Technik bekannte Nachteile weitestgehend eliminiert werden können.

Diese Aufgabe wird mit einem Mikroplattenreader umfassend eine Inkubationsvorrichtung gelöst, wie diese im Anspruch 1 definiert werden. Der erfindungsgemässe Mikroplatten-Reader umfasst zumindest:
a) einen Messraum;
b) eine Aktionsquelle, die zum Herbeiführen einer Wechselwirkung mit biologischen Strukturen in Wells einer Mikroplatte und zum Hervorrufen oder Erzeugen eines messbaren Signals ausgebildet ist;
c) eine Messeinrichtung, die zum Erfassen eines Signals ausgebildet ist, das durch biologische Strukturen in Wells einer Mikroplatte ausgesendet oder das durch die Aktionsquelle in oder an biologischen Strukturen in Wells einer Mikroplatte hervorgerufen oder erzeugt wurde; wobei die Messeinrichtung eine optische Achse definiert;
d) eine zumindest teilweise aus dem Messraum ausfahrbare Transportauflage, die zum Positionieren von Wells einer Mikroplatte gegenüber der optischen Achse der Messeinrichtung des Mikroplatten-Readers ausgebildet ist, indem die Transportauflage innerhalb des Messraums in zumindest einer Richtung bewegbar ausgebildet ist; und
e) eine Steuerung, die zum Steuern der Aktionsquelle, der Messeinrichtung sowie der Bewegungen der Transportauflage des Mikroplatten-Readers ausgebildet ist.

Der erfindungsgemässe Mikroplatten-Reader ist dadurch gekennzeichnet, dass er eine Inkubationsvorrichtung zum Reduzieren einer Flüssigkeits-Verdunstung aus Wells einer Mikroplatte umfasst, wobei:
(i) die Inkubationsvorrichtung einen Rahmen zum Aufnehmen einer Mikroplatte mit Wellböden aufweisenden Wells umfasst;
(ii) der Rahmen eine von einer inneren Wand umgebene, erste Öffnung umfasst, deren Masse zum Einlegen einer Mikroplatte ausgelegt sind; und
(iii) der Rahmen eine im Wesentlichen parallel zur inneren Wand verlaufende äussere Wand umfasst, die über einen Zwischenboden an der inneren Wand anschliesst, so dass durch die beiden Wände und den Zwischenboden ein die erste Öffnung umgebender Kanal zum Aufnehmen einer dem Inhalt der Mikroplattenwells angepassten Flüssigkeit gebildet ist.

Der erfindungsgemässe Mikroplatten-Reader ist zudem dadurch gekennzeichnet, dass die Inkubationsvorrichtung eine Tragfläche mit einer zweiten Öffnung umfasst, wobei diese Tragfläche an der inneren Wand und zum Tragen einer eingelegten Mikroplatte ausgebildet ist, und wobei infolge der zweiten Öffnung zumindest ein Teil der Böden der Wells einer in die Inkubationsvorrichtung eingelegten Mikroplatte durch die zweite Öffnung frei zugänglich sind.

Zudem ist der erfindungsgemässe Mikroplatten-Reader dadurch gekennzeichnet, dass die Transportauflage des Mikroplatten-Readers zumindest eine zum Auflegen oder Abheben der Inkubationsvorrichtung ausgebildete Auflagefläche mit einer Leseöffnung umfasst; und dass die Leseöffnung der Transportauflage in ihrer Grösse und Position der zweiten Öffnung des Rahmens der Inkubationsvorrichtung so angepasst ist, dass alle Böden der Wells einer in die Inkubationsvorrichtung eingelegten Mikroplatte durch die Leseöffnung frei zugänglich sind.

Des weiteren wird die Verwendung des Mikroplatten-Readers nach Anspruch 7 beansprucht.

Weitere bevorzugte und erfinderische Merkmale des Mikroplatten-Readers und dessen Verwendung ergeben sich jeweils aus den abhängigen Ansprüchen.

Vorteile des erfindungsgemässen Mikroplatten-Readers umfassen:
- Der Inkubationsrahmen bzw. die Inkubationskassette sind so dimensioniert und ausgestaltet, dass diese einfach (manuell oder robotisiert) in die Transportauflage eines Mikroplatten-Readers eingesetzt und ebenso einfach (manuell oder robotisiert) wieder von dieser Transportauflage entfernt werden können.
- Das Fixieren der Mikroplatte in einer definierten Position auf der Transportauflage des Mikroplatten-Readers und/oder im Inkubationsrahmen bzw. im Rahmen der Inkubationskassette wird durch einen bewährten Klemm-Mechanismus bewerkstelligt.
- Zumindest ein Teil der Böden, vorzugsweise aber alle Böden der Wells einer in den Inkubationsrahmen bzw. in die die Inkubationskassette eingelegten Mikroplatte bleiben von unten, also durch den Inkubationsrahmen bzw. den Rahmen der Inkubationskassette und durch die Leseöffnung der Transportauflage hindurch frei zugänglich, so dass der an sich bekannte Bottom-Reading-Modus des Mikroplatten-Readers angewendet werden kann.
- Der Inkubationsrahmen bzw. in die Inkubationskassette stellt ein mit Flüssigkeit (z.B. Wasser) befüllbares Reservoir in der Form eines Kanals bereit, der eine eingelegte Mikroplatte umgibt, und der die Atmosphäre in unmittelbarer Umgebung der Mikroplattenwells entsprechend anreichert.
- Der Kanal kann mittels einer Hand-Pipette, einer Pipette einer Labor-Arbeitsstation oder auch mittels eines Injektors des Mikroplatten-Readers zumindest teilweise mit einer Flüssigkeit angefüllt bzw. nachgefüllt werden.
- Um die angereicherte Atmosphäre von der Umgebung abzuschirmen kann ein Deckel auf den Inkubationsrahmen aufgelegt werden, wodurch eine Inkubationskassette geschaffen wird; dieser Deckel kann vom Mikroplatten-Reader selbst abgehoben und wieder aufgesetzt werden (vgl. die europäische Patentanmeldung EP 13 178 313.6, publiziert als EP 2 696 205 A1), so dass die Mikroplattenwells für die Zeit aller notwendigen Aktionen frei zugänglich sind.
- Die Verdunstungsrate der Flüssigkeit aus den Wells einer in die Inkubationskassette eingesetzten und zugedeckten Mikroplatte ist gegenüber einer konventionell zugedeckten Mikroplatte ohne Inkubationskassette deutlich reduziert.
- Der für Zellkulturen bzw. für Zell-basierte Versuche notwendige Gasaustausch zwischen den Mikroplattenwells und der Umgebung kann mittels sporadischem Abheben des Deckels der Inkubationskassette durch das Bedienungspersonal, einen Roboter oder durch den Mikroplatten-Reader selbst (vgl. die europäische Patentanmeldung Nr. 13 178 313.6, publiziert als EP 2 696 205 A1) unterstützt werden.
- Wird beispielsweise ein Inkubationsrahmen ohne Deckel in einem entsprechend optimierten Mikroplatten-Reader mit kleinem Messraum-Volumen verwendet, so wird die Gasatmosphäre im Messraum durch die Flüssigkeit im die eingelegte Mikroplatte umgebenden Reservoir entsprechend anreichert.
- In den Inkubationsrahmen bzw. in die Inkubationskassette können beliebige Mikroplatten eingelegt werden, wobei Standard-Mikroplatten gemäss ANSI_SBS 1-2-3-4-2004 Standard bevorzugt sind. Damit wird zum Durchführen von Versuchen mit Zellkulturen bzw. von Zell-basierten Versuchen das Verwenden von Spezial-Mikroplatten überflüssig.
- Dank der Verwendung eines Inkubationsrahmens bzw. einer Inkubationskassette können in einem Mikroplatten-Reader Langzeitversuche selbst bei erhöhter Temperatur (z.B. bei 37°C) durchgeführt werden.
- Im Verlauf von mehrtägigen Zellkulturexperimenten können bei der Verwendung einer Inkubationskassette bei temporär abgehobenem Kassettendeckel bzw. bei der Verwendung eines Inkubationsrahmens und unter Verwendung eines Injektors weitere Substanzen in die Wells der in die Inkubationskassette eingelegten Mikroplatte injiziert werden, um Reaktionen auszulösen oder zu stoppen bzw. um den Zellkulturen Nährstoffe oder andere, das Zellwachstum beeinflussende Substanzen zuzuführen.

Ein beispielhafter Mikroplatten-Reader mit Inkubationsrahmen bzw. Inkubationskassette wird anhand von schematischen Skizzen gezeigt. Diese Skizzen sollen ausgewählte Ausführungsformen des Erfindungsgegenstandes dokumentieren, aber den Umfang der vorliegenden Erfindung nicht einschränken. Dabei zeigt:
- Fig. 1: eine Ansicht einer Transportauflage eines Mikroplatten-Readers zum Transportieren einer Inkubationskassette, eines Inkubationsrahmens bzw. von Bestandteilen der Inkubationskassette und einer Standard-Mikroplatte gemäss ANSI_SBS 1-2-3-4-2004 Standard;
- Fig. 2: eine Ansicht eines Rahmens einer Inkubationskassette bzw. eines Inkubationsrahmens, der mit abgenommenem Deckel auf einer Transportauflage eines Mikroplatten-Readers liegt;
- Fig. 3: eine Ansicht eines Rahmens einer abgedeckten Inkubationskassette bzw. eines Inkubationsrahmens, in den mittels eines Roboters gerade eine Standard-Mikroplatte gemäss ANSI_SBS 1-2-3-4-2004 Standard eingelegt wird;
- Fig. 4: eine Ansicht eines Rahmens einer Inkubationskassette mit eingelegter Standard-Mikroplatte gemäss ANSI_SBS 1-2-3-4-2004 Standard, auf den gerade ein Deckel aufgelegt wird;
- Fig. 5: vertikale Teilschnitte durch eine zugedeckte Inkubationskassette, wobei:
Fig. 5A eine umlaufende Spalte;
Fig. 5B einen von mehreren abgesenkten Bereichen als Verbindung zwischen Kanal und erster Öffnung;
Fig. 5C einen ersten alternativen Deckel auf einem Rahmen einer Inkubationskassette gemäss Fig. 5A; und
Fig. 5D einen zweiten alternativen Deckel auf einem alternativen Rahmen zu Fig. 5B zeigt;
- Fig. 6: einen vertikalen Schnitt durch einen Mikroplatten-Reader mit kleinem Messraum-Volumen, in welchen eine Transportauflage mit Inkubationsrahmen und Mikroplatte eingezogen bzw. aus welchem eine Transportauflage mit Inkubationsrahmen und Mikroplatte ausgefahren wird.

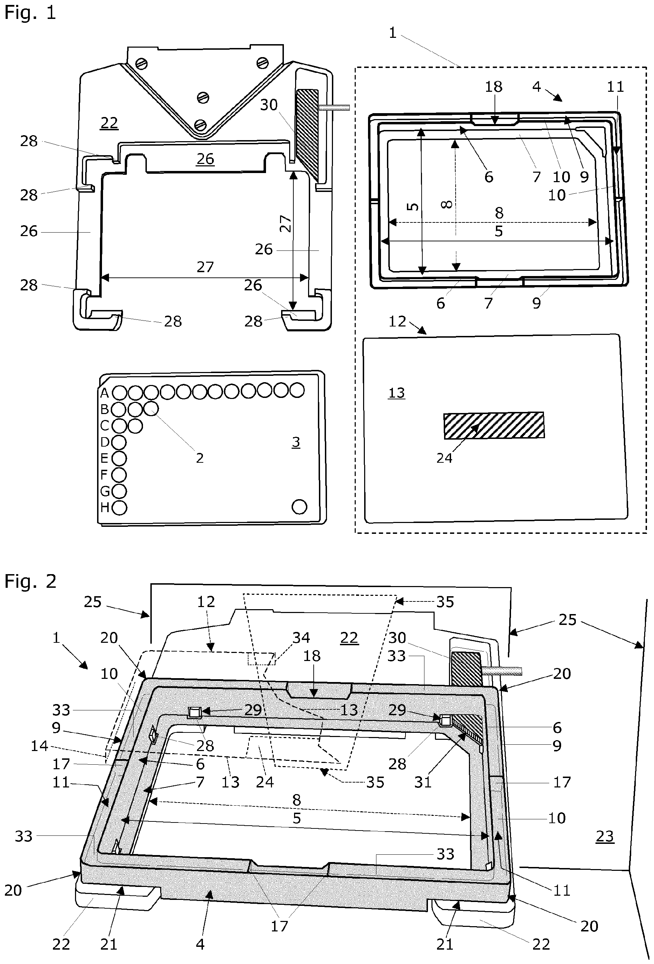

Die Figur 1 zeigt eine Ansicht von Bestandteilen einer Inkubationsvorrichtung des erfindungsgemässen Mikroplatten-Readers in Form einer Inkubationskassette 1, eine Transportauflage 22 eines Mikroplatten-Readers 23 zum Transportieren einer Inkubationskassette 1 und eine Standard-Mikroplatte 3 gemäss dem vom American National Standards Institute (ANSI) veröffentlichten SBS-Standard. Die dargestellte Mikroplatte 3 umfasst 96 in einem 8 x 12 Array angeordnete, Wellböden umfassende Wells 2, von denen nicht alle gezeichnet sind. Die 8 Reihen à 12 Wells 2 sind mit den Buchstaben A-H bezeichnet (vgl. ANSI_SBS 1-2-3-4-2004; 2006).

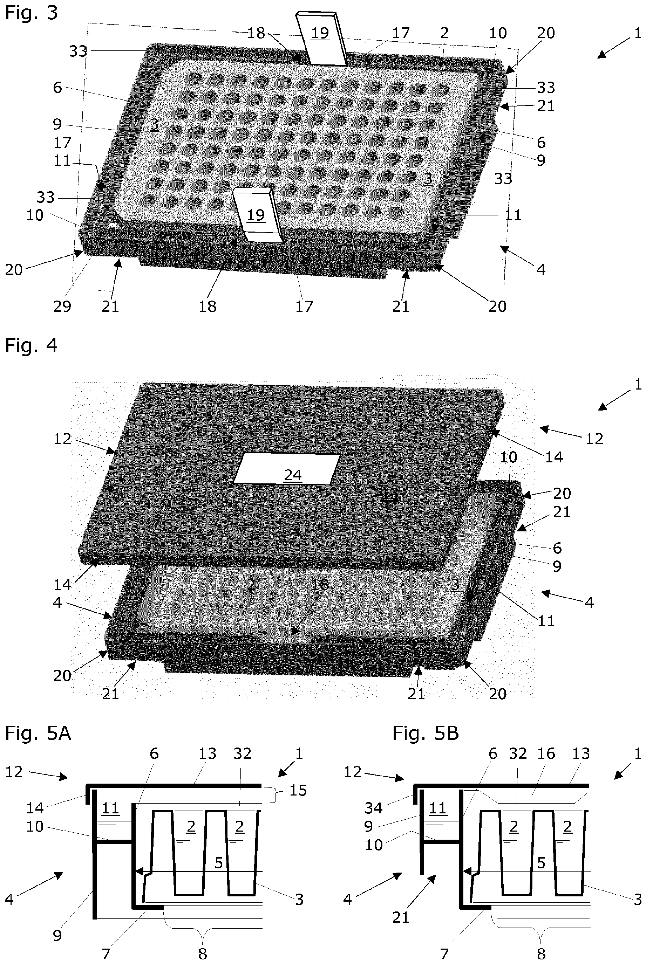

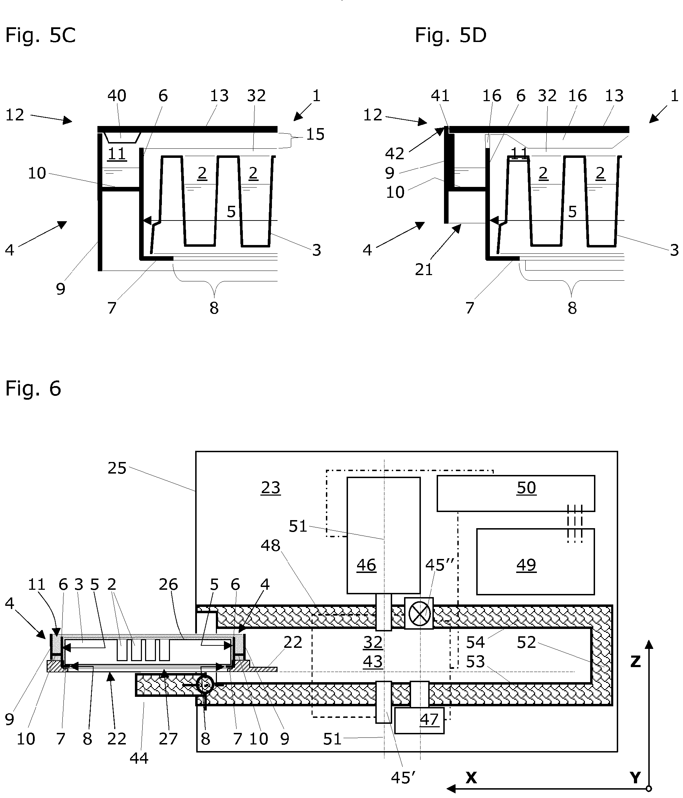

Die Inkubationskassette 1 ist zum Reduzieren der Flüssigkeits-Verdunstung aus Wells 2 von Mikroplatten 3 ausgebildet und umfasst bevorzugt einen Rahmen 4 und einen Deckel 12 (vgl. den gestrichelt eingerahmten Bereich in Fig. 1). Alternativ kann der Rahmen 4 als deckelloser Inkubationsrahmen 4, also ohne Deckel 12 verwendet werden (vgl. Fig. 6). Die Inkubationskassette 1 zum Reduzieren der Flüssigkeits-Verdunstung aus Wells 2 von Mikroplatten 3 umfasst einen vorzugsweise im Wesentlichen rechteckigen Rahmen 4 zum Aufnehmen einer Mikroplatte 3 (vorzugsweise einer Mikroplatte gemäss ANSI_SBS 1-2-3-4-2004 Standard) mit Flüssigkeit enthaltenden Wells 2. Der Rahmen 4 bzw. der Inkubationsrahmen 4 umfasst eine zentrale erste Öffnung 5, deren Masse zum vollständigen Einlegen einer Mikroplatte 3 ausgelegt sind. Dabei ist die zentrale erste Öffnung 5 von einer vorzugsweise im Wesentlichen senkrechten inneren Wand 6 umgeben, wobei bevorzugt an deren unterem Ende eine vorzugsweise im Wesentlichen horizontale Tragfläche 7 mit einer zentralen zweiten Öffnung 8 angeordnet ist. Diese Tragfläche 7 ist zum Tragen einer eingelegten Mikroplatte 3 ausgebildet. Der Rahmen 4 der Inkubationskassette 1 bzw. der Inkubationsrahmen 4 umfasst zudem eine bevorzugt im Wesentlichen parallel zur inneren Wand 6 verlaufende äussere Wand 9, die über einen Zwischenboden 10 an der inneren Wand 6 anschliesst, so dass durch die beiden Wände 6,9 und den Zwischenboden 10 ein die zentrale erste Öffnung 5 umgebender Kanal 11 zum Aufnehmen einer dem Inhalt der Mikroplattenwells 2 angepassten Flüssigkeit gebildet ist. Der Deckel 12 der Inkubationskassette 1 dient zum Abdecken des Rahmens 4 mit eingelegter Mikroplatte 3 und umfasst eine im Wesentlichen flache Platte 13 und vorzugsweise angeformt an diese Platte 13 einen nach unten abstehenden und umlaufenden Rand 14 bzw. mehrere nach unten abstehende Randteile 34 (vgl. Fig. 2). Der Deckel 12 ist beispielsweise so zum Auflegen auf den Rahmen 4 ausgebildet, dass die Platte 13 auf einem oberen Ende der äusseren Wand 9 aufliegt und dieser Rand 14 bzw. die Randteile 34 des Deckels 12 über die äussere Wand 9 des Rahmens 4 nach unten greifen (vgl. Fig. 2, 5A und 5B). Alternative Deckelausbildungen sind in den Figuren 5C und 5D dargestellt.

Der Deckel 12 umfasst vorzugsweise eine zumindest teilweise metallisierte, magnetisierbare Oberfläche 24, wobei diese magnetisierbare Oberfläche 24 ausgewählt ist aus einer Gruppe, die eine selbstklebende Metall-Folie, eine umspritzte Metall-Platte und eine angeklebte Metall-Platte umfasst, und wobei das Metall Eisen, Nickel und deren Legierungen umfasst. Der Deckel 12 ist vorzugsweise aus einem chemisch inerten Kunststoff und z.B. mittels Spritzgiessen herstellt. Der Deckel 12 kann eine Dichtung umfassen, welche die äussere Wand 9 des Rahmens 4 im Bereich ihres oberen Endes dichtend beaufschlägt (nicht gezeigt). Ebenso oder alternativ dazu kann die äussere Wand 9 des Rahmens 4 im Bereich ihres oberen Endes eine Dichtung umfassen, welche den Deckel 12 dichtend beaufschlägt (nicht gezeigt).

Die Fig. 1 zeigt zudem eine Transportauflage 22 eines Mikroplatten-Readers 23 (vgl. Fig. 2 und 6), wobei die Transportauflage 22 eine Auflagefläche 26 zum Auflegen eines Rahmens 4 einer Inkubationskassette 1 bzw. eines Inkubationsrahmens 4 umfasst. Falls mehrere Mikroplatten 3 parallel bearbeitet werden sollen wird bevorzugt, dass die Transportauflage 22 zwei oder mehr Auflageflächen 26 zum Auflegen je eines Rahmens 4 (also von zwei oder mehr Rahmen 4) der erfindungsgemässen Inkubationskassette 1 bzw. Inkubationsrahmen 4 umfasst. Eine Transportauflage 22 kann (wie in Fig. 1 gezeigt) mehrere Auflageflächen 26 zum Auflegen von nur einer Inkubationskassette 1 bzw. eines Inkubationsrahmens 4 umfassen. Die Transportauflage 22 kann vorzugsweise zum optischen Analysieren der Proben in den Wells 2 einer Mikroplatte 3 und/oder zum Abheben/Auflegen eines Deckels 12 von bzw. auf die Inkubationskassette 1 und/oder zum Zugeben von Substanzen zu den Wells 2 der Mikroplatte 3 und/oder zum Kanal 11 der Inkubationskassette 1 bzw. des Inkubationsrahmens 4 innerhalb des Gehäuses 25 des Mikroplatten-Readers 23 in zumindest einer horizontalen Richtung, z.B. in einer X-Richtung eins kartesischen Koordinatensystems (vgl. Fig. 6) bewegt werden. Die Transportauflage 22 kann zum Auflegen oder Abheben einer Inkubationskassette 1 bzw. eines Inkubationsrahmens 4 zudem in einen offenen Bereich des Mikroplatten-Readers 23 oder sogar über den Mikroplatten-Reader hinaus in einer horizontalen Richtung bewegt werden. Der Mikroplatten-Reader ist bevorzugt zum Steuern und Ausführen dieser Bewegungen der Transportauflage 22 sowie zum Lösen und/oder Anpressen des Federriegels 30 ausgebildet.

Vorzugsweise umfasst die Auflagefläche 26 der Transportauflage 22 eine im Wesentlichen rechteckige Leseöffnung 27, die in ihrer Grösse und Position der zentralen zweiten Öffnung 8 des Rahmens 4 der erfindungsgemässen Inkubationskassette 1 bzw. des Inkubationsrahmens 4 angepasst ist. Es wird besonders bevorzugt, dass die Auflagefläche 26 an den Ecken der Leseöffnung 27 angeordnete Haltestege 28 umfasst (5 von total 8 gezeichneten Haltestegen sind hier mit 28 bezeichnet), die zum Eingreifen in entsprechende Halteöffnungen 29 in der inneren Wand 6 des Rahmens 4 der Inkubationskassette 1 bzw. des Inkubationsrahmens 4 (grau markiert in Fig. 2) ausgebildet sind. Vorzugsweise umfasst die Transportauflage 22 einen an sich bekannten Federriegel 30 zum Positionieren und Halten einer in den Rahmen 4 der Inkubationskassette 1 eingelegten Mikroplatte 3.

Die Figur 2 zeigt eine Ansicht eines Rahmens 4 einer Inkubationskassette 1 bzw. eines Inkubationsrahmens 4, der mit abgenommenem Deckel 12 (deshalb hier gestrichelt angedeutet) auf einer Transportauflage 22 eines Mikroplatten-Readers 23 liegt. Dieser Deckel 12 kann einen umlaufenden Rand 14 (vgl. linke Seite) oder eine Anzahl Randteile 34 (siehe hinten) umfassen, wobei die Randteile 34 im Bereich der Ecken 20 des Rahmens 4 über die äussere Wand 9 nach unten greifend ausgebildet sein können. Alternativ oder zusätzlich können Randteile 34 vorgesehen sein, die zwischen den Ecken 20 über die äussere Wand 9 nach unten greifend ausgebildet sind. Der umlaufende Rand 14 kann zusätzlich zum zentrierten Platzieren auf und zum Verhindern des Abrutschens des Deckels 12 von dem Rahmen 4 eine Dichtfunktion zwischen der Gasatmosphäre 32 im Innern der Inkubationskassette 1 (vgl. Fig. 5A) ausüben. Die Randteile 34 können dagegen kaum eine solche Dichtfunktion ausüben. Alternative Deckelausbildungen und entsprechende Deckelauflagen sind in den Figuren 5C und 5D dargestellt.

Bei der hier gezeigten Inkubationskassette 1 bzw. dem hier gezeigten Inkubationsrahmen 4 reicht die innere Wand 6 des Rahmens 4 etwa gleich hoch wie die äussere Wand 9, in einem solchen Fall wird jedoch bevorzugt, dass die innere Wand 6 abgesenkte Bereiche 16 umfasst, so dass bei aufgelegtem Deckel 12 jeder abgesenkte Bereich 16 die zentrale erste Öffnung 5 mit dem diese umgebenden Kanal 11 verbindet (vgl. Fig. 5B und 5D). Alternativ kann vorgesehen sein, dass die innere Wand 6 des Rahmens 4 weniger hoch reicht als die äussere Wand 9, so dass bei aufgelegtem Deckel 12 eine umlaufende Spalte 15 die zentrale erste Öffnung 5 mit dem diese umgebenden Kanal 11 verbindet (vgl. Fig. 5A und 5C). Zudem kann vorgesehen werden, dass (wie in Fig. 2 gezeigt) die innere Wand 6 des Rahmens 4 über im Wesentlichen senkrechte Stege 17 mit der äusseren Wand 9 des Rahmens 4 verbunden ist, wodurch der Kanal 11 bzw. das Reservoir 11 in eine Anzahl Kanalabschnitte 33 unterteilt wird.

Es wird bevorzugt, dass die innere Wand 6 des Rahmens 4 einer Inkubationskassette 1 bzw. eines Inkubationsrahmens 4 Ausschnitte 18 (vgl. Fig. 1 und 2) umfasst, die zum Eingreifen von Fingern 19 eines Mikroplatten-Handling-Roboters (in Fig. 3 gezeigt) oder einer Bedienungsperson (nicht gezeigt) zum Transportieren einer Mikroplatte 3 (vorzugsweise einer Mikroplatte 3 gemäss ANSI_SBS 1-2-3-4-2004 Standard) ausgebildet sind. Der Rahmen 4 ist vorzugsweise aus einem chemisch inerten Kunststoff und z.B. mittels Spritzgiessen herstellt.

Vorzugsweise umfasst der die zentrale erste Öffnung 5 umgebende Kanal 11 ein wasserbindendes Material, das bei Temperaturen von mindestens 35 °C, bevorzugt von mindestens 25 °C, Wasserdampf an eine über den Wells 2 der Mikroplatte 3 bestehende Gasatmosphäre 32 (vgl. Fig. 5A und 5B) abgibt. Speziell als wasserbindendes Material bevorzugt wird ein quellbares Polyacrylat (vgl. weiter unten).

Um einen sicheren Sitz auf der Transportauflage 22 des Mikroplatten-Readers 23 zu erreichen umfasst die äussere Wand 9 des Rahmens 4 der Inkubationskassette 1 bzw. des Inkubationsrahmens 4 an allen Ecken 20 Einschnitte 21, die zum Auflegen des Rahmens 4 mit oder ohne eine in diesen Rahmen 4 bzw. Inkubationsrahmen 4 eingelegte Mikroplatte 3 (vorzugsweise gemäss dem ANSI_SBS 1-2-3-4-2004 Standard) auf eine Transportauflage 22 eines Mikroplatten-Readers 23 ausgebildet sind. Zudem wird bevorzugt, dass die Auflagefläche 26 an den Ecken der Leseöffnung 27 angeordnete Haltestege 28 umfasst, die zum Eingreifen in entsprechende Halteöffnungen 29 in der inneren Wand 6 des Rahmens 4 der Inkubationskassette 1 bzw. des Inkubationsrahmens 4 ausgebildet sind. Trotz dieses sicheren Sitzes, der insbesondere während dem Verfahren der Transportauflage 22 in den Mikroplatten-Reader hinein oder daraus heraus bzw. beim Abrastern der Wells im Mikroplatten-Reader wichtig und unentbehrlich ist, kann die ganze Inkubationskassette 1 mit Mikroplatte 3 und Deckel 12 oder auch nur der Inkubationsrahmen 4 einfach in einer vertikalen Linearbewegung auf die Transportauflage 22 gelegt oder davon abgehoben werden.

Der Federriegel 30 der Transportauflage dient zum Positionieren und Halten einer in den Rahmen 4 der Inkubationskassette 1 bzw. in den Inkubationsrahmen 4 eingelegten Mikroplatte 3 und umfasst vorzugsweise eine schiefe Anschlagfläche 31, die zum Beaufschlagen einer Ecke einer in den Rahmen 4 der Inkubationskassette 1 eingelegten Mikroplatte 3 ausgebildet ist. Dieses Beaufschlagen bewirkt, dass die Mikroplatte 3 gegen die der schiefen Anschlagfläche 31 gegenüber liegenden Haltestege 28 der Transportauflage 22 gedrückt und damit exakt auf der Transportauflage 22 positioniert wird. Soll also eine Mikroplatte 3 aus einem auf der Transportauflage 22 liegenden Rahmen 4 genommen werden, so muss der Federriegel 30 zum Freigeben der Mikroplatte 3 von dieser wegbewegt werden. Falls ein Rahmen 4 bzw. Inkubationsrahmen 4 mit eingelegter Mikroplatte 3 von der Transportauflage 22 abgehoben werden soll, muss ebenfalls der Federriegel 30 zum Freigeben der Mikroplatte 3 von dieser wegbewegt werden.

Die Figur 3 zeigt eine 3D-Ansicht eines Rahmens 4 einer abgedeckten Inkubationskassette 1 bzw. eines Inkubationsrahmens 4, in den mittels eines Roboters gerade eine Mikroplatte 3 eingelegt wird; das Entfernen der Mikroplatte 3 aus dem Rahmen 4 bzw. aus dem Inkubationsrahmen 4 würde genauso aussehen. Zum einfacheren Ausführen eines derartigen Einlegens oder Entfernens wird bevorzugt, dass die innere Wand 6 des Rahmens 4 Ausschnitte 18 (vgl. auch Fig. 1 und 2) umfasst, die zum Eingreifen von Fingern 19 eines Mikroplatten-Handling-Roboters (gezeigt) oder einer Bedienungsperson (nicht gezeigt) zum Transportieren einer Mikroplatte 3 (vorzugsweise einer Standard-Mikroplatte 3 gemäss ANSI_SBS 1-2-3-4-2004 Standard) ausgebildet sind. Im Übrigen ist hier derselbe Rahmen 4 der Inkubationskassette 1 bzw. derselbe Inkubationsrahmen 4 wie in Fig. 2, aber eben mit eingesetzter Mikroplatte 3 abgebildet, so dass sich die nochmalige Beschreibung der mit Bezugszeichen versehenen Details erübrigt.

Die Figur 4 zeigt eine 3D-Ansicht eines Rahmens 4 einer Inkubationskassette 1 mit eingelegter Standard-Mikroplatte 3, auf den gerade ein Deckel 12 aufgelegt wird, das Abheben des Deckels 12 von dem Rahmen 4 würde genau so aussehen. Das Auflegen oder Abheben des Deckels 12 kann ausserhalb des Gehäuses 25 des Mikroplatten-Readers 23 manuell oder mit einem Roboter erfolgen. Innerhalb des Gehäuses 25 erfolgt diese Manipulation am Deckel 12 der Inkubationskassette 1 bevorzugt mittels einer in den Mikroplatten-Reader 23 integrierten Magnetvorrichtung 35 (vgl. Fig. 2). Eine bevorzugte Magnetvorrichtung 35 mit Permanentmagneten wird in der europäischen Patentanmeldung EP 13178313.6 des aktuellen Patentanmelders beschrieben. Der abgebildete Deckel 12 umfasst eine Platte 13 mit einer magnetisierbaren Oberfläche 24, welche lediglich einen Teil der Platte 13 bedeckt; alternativ könnten mehrere derartig kleine magnetisierbare Oberflächen 24 oder eine einzige grosse magnetisierbare Oberfläche 24 vorgesehen sein, welche zumindest annähernd die ganze Platte 13 abdeckt. Der hier dargestellte, bevorzugte Deckel 12 umfasst einen umlaufenden Rand 14. Im Übrigen ist hier derselbe Rahmen 4 der Inkubationskassette 1 wie in Fig. 2, aber eben mit eingesetzter Mikroplatte 3 abgebildet, so dass sich die nochmalige Beschreibung der mit Bezugszeichen versehenen Details erübrigt.

Die Figur 5 umfasst die Fig. 5A bis 5D und zeigt schematische vertikale Teilschnitte durch eine mit einem Deckel 12 zugedeckte Inkubationskassette 1 mit einer in die zentrale erste Öffnung 5 des Rahmens 4 eingesetzten Mikroplatte 3, welche auf einer Tragfläche 7 und über der zentralen zweiten Öffnung 8 ruht. Die Tragfläche 7 ist mit der inneren Wand 6 verbunden, welche wiederum über den Zwischenboden 10 mit der äusseren Wand 9 verbunden ist. Die äussere Wand 9, der Zwischenboden 10 und die innere Wand 6 definieren den Kanal 11 bzw. das Feuchtreservoir in welchem eine der Flüssigkeit in den Wells 2 der Mikroplatte 3 angepasste Flüssigkeit (z.B. destilliertes Wasser) eingefüllt ist.

Gemäss der Fig. 5A reicht die innere Wand 6 des Rahmens 4 weniger hoch als die äussere Wand 9, wobei bei aufgelegtem Deckel 12 eine umlaufende Spalte 15 zwischen der Platte 13 und dem oberen Ende der inneren Wand 6 die zentrale erste Öffnung 5 mit dem diese umgebenden Kanal 11 verbindet. Damit entsteht über dem Reservoir oder Kanal 11 und über den Wells 2 der Mikroplatte 3 eine zusammenhängende Gasatmosphäre 32. Der Deckel 12 umfasst hier einen die Platte 13 vollständig umlaufenden Rand 14.

Gemäss der Fig. 5B reicht die innere Wand 6 des Rahmens 4 gleich hoch wie die äussere Wand 9, wobei die innere Wand 6 abgesenkte Bereiche 16 umfasst, so dass bei aufgelegtem Deckel 12 jeder abgesenkte Bereich 16 die zentrale erste Öffnung 5 mit dem diese umgebenden Kanal 11 verbindet. Damit entsteht über dem Reservoir oder Kanal 11 und über den Wells 2 der Mikroplatte 3 eine zusammenhängende Gasatmosphäre 32. Dargestellt ist einer von mehreren abgesenkten Bereichen als Verbindung zwischen Kanal 11 und erster Öffnung 5. Der Deckel 12 umfasst hier einen Randteil 34 der nur einem Teil des in Fig. 5A vollständig umlaufenden Randes 14 entspricht und hier so ausgebildet ist, dass er im Bereich einer Ecke 20 die äussere Wand 9 nach unten übergreift. Aus diesem Grund ist hier auch der entsprechende Einschnitt 21 in der äusseren Wand 9 im Bereich der Ecke 20 dargestellt.

Gemäss der Fig. 5C reicht die innere Wand 6 des Rahmens 4 weniger hoch als die äussere Wand 9, wobei bei aufgelegtem Deckel 12 eine umlaufende Spalte 15 zwischen der Platte 13 und dem oberen Ende der inneren Wand 6 die zentrale erste Öffnung 5 mit dem diese umgebenden Kanal 11 verbindet. Damit entsteht über dem Reservoir oder Kanal 11 und über den Wells 2 der Mikroplatte 3 eine zusammenhängende Gasatmosphäre 32 (vgl. auch Fig. 5A). Der Deckel 12 ist hier als einfache, randlose Platte 13 ausgebildet und liegt auf der äusseren Wand 9 auf. Um ein korrektes Auflegen des Deckels 12 zu erleichtern und um ein Verrutschen eines aufgelegten Deckels 12 auf dem Rahmen 4 zu verhindern, weist dieser Deckel 12 an seiner Unterseite Zentriernocken 40 auf. Diese Zentriernocken 40 können aus dem gleichen Material wie der Deckel 12 bestehen und zusammen mit dem Deckel spritzgegossen oder an den Deckel 12 geklebt oder geschweisst sein. Auch kann der Deckel 12 an seiner Unterseite oder die äussere Wand an deren höchster Stelle eine umlaufende Dichtung aufweisen (beides nicht gezeigt), welche die Abdichtung der Inkubationskassette 1 zusätzlich verbessert.

Gemäss der Fig. 5D reicht die innere Wand 6 des Rahmens 4 praktisch gleich hoch wie die äussere Wand 9, wobei die innere Wand 6 abgesenkte Bereiche 16 umfasst, so dass bei aufgelegtem Deckel 12 jeder abgesenkte Bereich 16 die zentrale erste Öffnung 5 mit dem diese umgebenden Kanal 11 verbindet. Damit entsteht über dem Reservoir oder Kanal 11 und über den Wells 2 der Mikroplatte 3 eine zusammenhängende Gasatmosphäre 32. Dargestellt sind zwei von mehreren abgesenkten Bereichen 16 als Verbindung zwischen Kanal 11 und erster Öffnung 5 (vgl. auch Fig. 5B). Die äussere Wand 9 ist hier in deren oberem Bereich verdickt und weist einen Absatz 41 auf, welcher vom Deckel 12 beaufschlagt wird. Der Deckel 12 ist hier als einfache Platte ausgebildet und wird von einem hochragenden Teil 42 der äusseren Wand 9 am Verrutschen gehindert. Der Deckel 12 kann an seiner Unterseite oder der Absatz 41 und/oder die Innenfläche des hochragenden Teils 42 der äusseren Wand 9 können eine umlaufende Dichtung aufweisen (beides nicht gezeigt), welche die Abdichtung der Inkubationskassette 1 zusätzlich verbessert.

Einige, mit einer eingelegten 96-Well Flachboden Mikroplatte 3 gemäss ANSI_SBS 1-2-3-4-2004 Standard bestückte Inkubationskassetten 1 wurden auf die Transportauflage 22 eines Mikroplatten-Readers 23 gelegt. Die Wells 2 dieser Mikroplatten 3 waren mit je einer wässerigen Flüssigkeitsprobe mit einem Volumen von 200 µl versehen. In den die zentrale erste Öffnung 5 umgebenden Kanal 11 der Inkubationskassetten 1 wurde ein jeweils dem Gesamtvolumen der Flüssigkeitsproben in den Wells 2 der Mikroplatten 3 in etwa entsprechendes Volumen von ca. 10 ml destilliertem Wasser eingefüllt bzw. auf die Kanalabschnitte 33 verteilt.

Bei einer Inkubationskassette 1, bei welcher der Kanal 11 nicht durch Stege 17 unterteilt war wurde beobachtet, dass schon beim routinemässigen Verfahren der Transportauflage 22 im Mikroplatten-Reader 23 Wasser aus dem Kanal 11 durch Überschwappen austritt. Bei einer Inkubationskassette 1, bei welcher der Kanal 11 gemäss der Fig. 2 durch Stege 17 in Kanalabschnitte 33 unterteilt war, wurde beobachtet, dass sie weniger anfällig auf ein derartiges Überschwappen des Wassers war. Allerdings besteht z.B. beim Schütteln einer sich in der Inkubationskassette 1 befindlichen Mikroplatte 3, d.h. heisst beim Agitieren der Flüssigkeitsproben in den Wells 2 dieser Mikroplatte auf einem Schüttelgerät eine wesentlich grössere Gefahr des Überschwappens von Flüssigkeit aus dem Kanal 11 bzw. aus den Kanalabschnitten 33. Zu keiner Zeit wurde ein Überschwappen der Flüssigkeitsproben aus den Wells 2 der Mikroplatten 3 beobachtet.

Mit unterschiedlichsten Massnahmen wurde versucht dieses Überschwappen aus dem Kanal 11 zu verhindern:
1. Der Kanal 11 bzw. die Kanalabschnitte 33 wurden mit schwammartigem Material (z.B. Haushaltsschwamm, medizinisches Vlies) ausgekleidet. Diese Massnahme wird nicht bevorzugt, weil dieses schwammartige Material einerseits selbst ein gewisses Volumen benötigt und sich dadurch das nutzbare Füllvolumen reduziert. Andererseits fällt dieses schwammartige Material leicht aus dem Kanal 11 bzw. aus den Kanalabschnitten 33 heraus und kann verloren gehen. Eine Befestigung (z.B. Ankleben) des schwammartigen Materials im Flüssigkeitsreservoir, also im Kanal 11 bzw. in den Kanalabschnitten 33 ist sehr aufwändig und kann zusätzlich das nutzbare Füllvolumen reduzieren.
2. Das Vorsehen von einer Vielzahl von Stegen 17 und damit ein Unterteilen des Kanals 11 in immer kleinere Kanalabschnitte 33 kann ein Überschwappen erheblich reduzieren. Ein solcher Einbau von vielen Querstegen als "Wellenbrecher" stellt jedoch ebenfalls keine bevorzugte Lösung dar, weil das Verteilen des Flüssigkeitsvolumens im Kanal 11 aufwändiger wird, da sich das Wasser bei hohen Stegen nicht mehr von selbst im Reservoir verteilt. Bei einer Alternative mit so niedrigen Stegen, dass sich das Wasser noch genügend im Reservoir verteilt ist hingegen der erreichbare Effekt zu gering.
3. Die Erhöhung der Viskosität der Flüssigkeit im Reservoir bei zumindest annähernd gleichem Verdunstungsverhalten erscheint als die vielversprechendste Lösung. Vorstellbar ist z.B. die Gelbildung mit Hilfe von Gelatine, Agarose-Gel, Methylzellulose, usw. oder ein Zumischen von Flüssigkeiten hoher Viskosität, wie z.B. Glycerin, Glykol, usw.

Für die Erhöhung der Viskosität der Flüssigkeit im Reservoir sind allerdings einige Anforderungen zu beachten:
- Der Gelbildner darf höchstens leicht hygroskopisch sein, um die Verdunstung der sich "opfernden" Reservoir-Flüssigkeit zu gewährleisten (ungeeignet sind dafür Glycerin oder Glykol);
- Das Gel muss leicht und ohne Aufwand herstellbar sein (ungeeignet sind hier Gelatine- und Agarose-Gele, die erst gekocht werden müssen);
- Während der Benutzung im Mikroplatten-Reader eingetrocknete oder angetrocknete, mit einem Gel gefüllte Reservoirs müssen beim Wiederauffüllen (z.B. mittels eines Injektors) innerhalb kurzer Zeit wieder ihren gequollenen Zustand erreichen (ungeeignet sind Gelatine, Agarose und Methylzellulose);
- Das gelbildende Material soll als Nährmedium für Mikroorganismen ungeeignet sein; denn Zellkulturen in Mikroplatten sind anfällig für Pilz- und Bakterienbefall aus der Umgebung (ungeeignet sind Gelatine und Agarose-Gele);
- Die Kosten für den Gelbildner müssen möglichst niedrig sein;
- Der Gelbildner soll einen möglichst geringen Gewichtsanteil haben.

Mit Polyacrylsäure-Natriumsalz (z.B. von Sigma-Aldrich, Bestellnummer 436364) wurde ein Material gefunden, das in jeder Hinsicht ideal zum Einsatz als Gelbildner im Kanal 11 bzw. in den Kanalabschnitten 33 geeignet ist:
- Polyacrylsäure quillt innerhalb von 3 Minuten auf ein 200-faches seines Volumens auf, wobei beispielsweise 6 g/l Polyacrylat/Wasser ein brauchbares Gel ergeben;
- Das Wasser aus diesem Polyacrylat-Gel verdunstet praktisch gleich schnell wie reines Wasser: eine Probe destilliertes Wasser verlor beim Lagern während 7 Stunden in einem Wärmeschrank bei 50 °C etwa 68 % des Anfangsvolumens, während den gleichen Bedingungen verlor ein Gemisch (1.6 Gew.-% Polyacrylsäure in Wasser) etwa 60 % des Anfangsvolumens; das Wasser aus dem Polyacrylat-Gel verdunstet somit nur um ca. 8% langsamer als reines Wasser;
- Einmal eingetrocknet, quillt das Polyacrylat-Gel rasch wieder auf bei erneuter Wasserzugabe;
- Das Polyacrylsäure-Gel wird von allen Kleinlebewesen verschmäht (ein Polyacrylat-Gel wurde während drei Monaten offen stehen gelassen, ohne dass eine Besiedelung mit Mikroorganismen festgestellt werden konnte);
- Das Polyacrylsäure-Gel ist gesundheitlich unbedenklich und wird z.B. in der Pharmazie (vgl. EP 0 744 951 B1), zur Herstellung von Hygieneartikeln mit erhöhter Absorptionsfähigkeit für Körperflüssigkeiten (vgl. EP 1 137 678 B1) und im Gartenbau (vgl. WO 98/49252 A1) eingesetzt;
- Die benötigte Menge Polyacrylsäure pro Inkubationskassette 1 kostet lediglich Bruchteile eines Eurocents und verteuert das Herstellen einer Inkubationskassette 1 nur unwesentlich;
- Idealerweise werden Polyacrylat-Körner mit einer geringen Menge wasserlöslichem Kleber (z.B. Polyvinylalkohol, Polyvinylacetat, Polyvinylpyrrolidon) im Reservoir der Inkubationskassette 1 fixiert, dass der Anwender nur noch wie gewohnt Wasser einfüllen muss.

Ein in der Fig. 1 vorgestellter Deckel 12 kann vor dem Einfüllen der Flüssigkeit in den Kanal 11 bzw. in die Kanalabschnitte 33 einer auf der Transportauflage 22 aufgelegten Inkubationskassette 1 mit einer in das Gehäuse 25 des Mikroplatten-Readers 23 integrierten Magnetvorrichtung 35 (in Fig. 2 gestrichelt gezeigt), manuell oder mit einem Roboter einer Labor-Arbeitsstation (nicht gezeigt) vom Rahmen 4 der Inkubationskassette 1 abgehoben werden. Zusätzlich oder alternativ dazu kann ein solcherart ausgerüsteter Deckel 12 mit magnetisierbarer Oberfläche 24 nach dem Einfüllen der Flüssigkeit in den Kanal 11 bzw. in die Kanalabschnitte 33 einer auf der Transportauflage 22 aufgelegten Inkubationskassette 1 mit einer in das Gehäuse 25 des Mikroplatten-Readers 23 integrierten Magnetvorrichtung 35 (in Fig. 2 gestrichelt gezeigt), manuell oder mit einem Roboter einer Labor-Arbeitsstation (nicht gezeigt) auf den Rahmen 4 der Inkubationskassette 1 gelegt werden. Vorzugsweise umfasst der Mikroplatten-Reader 23 eine in das Gehäuse 25 integrierte Magnetvorrichtung 35 zum Abheben und Auflegen des Deckels 12 einer auf der Transportauflage 22 aufgelegten Inkubationskassette 1.

Der Kanal 11 bzw. die Kanalabschnitte 33 eines Rahmens bzw. eines Inkubationsrahmens 4 werden bevorzugt zumindest teilweise mit einer Flüssigkeit (z.B. mit destilliertem Wasser) gefüllt, wobei zum Einfüllen der Flüssigkeit in den Kanal 11 bzw. in die Kanalabschnitte 33 eine Hand-Pipette, eine Pipette einer Labor-Arbeitsstation oder ein Injektor des Mikroplatten-Reader 23 verwendet wird. Auf diese Weise wird das Verwenden der erfindungsgemässen Inkubationskassette 1 bzw. des Inkubationsrahmens 4 zum Reduzieren der Flüssigkeits-Verdunstung aus Wells 2 von Mikroplatten 3 in einem Mikroplatten-Reader 23 ermöglicht bzw. unterstützt. Falls Mikroplatten 3 gemäss einem anderen Standard als ANSI_SBS 1-2-3-4-2004 verwendet werden sollen, können die Dimensionen der Inkubationskassette 1 bzw. des Inkubationsrahmens 4 entsprechend angepasst werden.

Die Figur 6 zeigt einen Vertikalschnitt durch einen Mikroplatten-Reader 23 mit minimierten Messraum-Volumen beim Einziehen einer Mikroplatte 3 in den vorzugsweise als lichtdicht abschliessbare Kammer ausgebildeten Messraum 43. Dieser Mikroplatten-Reader 23, umfasst eine Transportauflage 22 zum Aufnehmen von zumindest einer Mikroplatte bzw. einer Standard-Mikroplatte 3 mit biologischen Strukturen enthaltenden Wells 2, von denen nur vier dargestellt sind.

Im Zusammenhang mit der vorliegenden Erfindung umfasst der Begriff "biologische Strukturen" Gewebeteile, z.B. von Menschen, Tieren oder Pflanzen; Zellkulturen oder Teile davon; Einzelzellen; Zellorganellen; Makromoleküle wie Nukleinsäuren oder Proteine sowie Einzelmoleküle wie Nukleotide, Aminosäuren, Hormone und Metaboliten.

Diese Transportauflage 22 ist vorzugsweise so weit aus dem Messraum 43 des Mikroplatten-Readers 23 ausfahrbar ausgebildet, dass zumindest ein Rahmen 4 einer Inkubationskassette 1 bzw. ein Inkubationsrahmen 4 und/oder eine Mikroplatte 3 von Hand oder mittels eines Mikroplatten-Handling-Roboters (beides nicht dargestellt) in diese Transportauflage 22 eingelegt bzw. davon abgehoben werden können. Die Transportauflage 22 ist hier bereits teilweise eingefahren, weil gerade eine Mikroplatte 3 und den diese umgebenden Inkubationsrahmen 4 in den Mikroplatten-Reader 23 eingeschoben werden. Während des Einschiebens oder Auswerfens einer Mikroplatte 3 und den diese umgebenden Inkubationsrahmens 4 ist vorzugsweise eine Klappe 44 geöffnet, welche im geschlossenen Zustand den Messraum 43 bevorzugt lichtdicht verschliesst, damit kein die Untersuchungen beeinflussendes Licht aus der Umgebung in den Messraum 43 gelangen kann. Neben dem Aufnehmen von zumindest einer Mikroplatte 3 und einen diese umgebenden Inkubationsrahmen 4 dient diese Transportauflage 22 zum Positionieren der Mikroplatte 3 mit den biologischen Strukturen (z.B. Metaboliten, Makromoleküle, Zellen oder Zellkulturen) enthaltenden Wells 2 gegenüber Aktionsquellen 45',45" und gegenüber Messeinrichtungen 46,47 des Mikroplatten-Readers 23 bzw. gegenüber den optischen Achsen 51 der Messeinrichtungen 46,47. Der abgebildete Mikroplatten-Reader 23 umfasst des Weiteren zumindest eine Aktionsquelle 45',45" zum Herbeiführen einer Wechselwirkung zwischen zumindest einer dieser Aktionsquellen 45',45" und biologischen Strukturen in bestimmten Wells 2 der Mikroplatte 3 und zum Hervorrufen oder Erzeugen eines messbaren Signals. Solche Signale umfassen Fluoreszenzemission, Lumineszenzemission, reflektiertes Licht und transmittiertes Licht.

In diesem Ausführungsbeispiel dient eine erste Lichtquelle als Aktionsquelle 45' zum Anregen einer Fluoreszenz in oder an biologischen Strukturen in Wells 2 dieser Mikroplatte 3 und eine erste Messeinrichtung 46 (hier in Form einer Photomultiplier-Röhre) wird zum sogenannten "Top Reading" der von den Proben ausgesendeten Fluoreszenz in Bezug auf eine optische Achse 51 verwendet. Alternativ kann die sowohl die Anregung von oben als auch die Detektion einer Fluoreszenz mittels Top Reading erfolgen. Soll hingegen die Lumineszenz von Proben im Top Reading detektiert werden, kann sogar auf eine Aktionsquelle verzichtet werden. Eine zweite Lichtquelle dient hier als Aktionsquelle 45" zum Beleuchten von biologischen Strukturen in Wells 2 dieser Mikroplatte 3 und eine zweite Messeinrichtung 47 (hier in Form einer Digitalkamera) wird zum sogenannten "Bottom Reading" der Absorbance der Proben in Bezug auf eine optische Achse 51 verwendet.

Derartige Lichtquellen sind beispielsweise ausgewählt aus einer Gruppe, die Lichtbogenlampen, Blitzlampen, Glühlampen (wie z.B. Halogenlampen), Laser, Laserdioden und Leuchtdioden (LEDs) umfasst. Die entsprechenden Wellenlängen zum Anregen der Fluoreszenz sowie die entsprechenden Fluorophore und deren Emissionscharakteristika sind dem Fachmann ebenfalls bekannt und werden je nach Anwendung ausgewählt. Auch das nicht-invasive Durchstrahlen von Zellen oder Zellkulturen zum Erfassen der Absorbance sowie die dazu zu verwendenden Lichtquellen sind jedem Fachmann geläufig. Messeinrichtungen 46,47 zum Erfassen zumindest eines integralen Signals, das durch die Aktionsquelle(n) 45',45" in oder an biologischen Strukturen in den bestimmten Wells 2 der Mikroplatte 3 hervorgerufen oder erzeugt wurde sind bevorzugt ausgewählt ist aus einer Gruppe, die Photomultiplier, Photodioden, Photodioden-Arrays und Avalanche-Dioden umfasst. Die Messeinrichtungen 46,47 und Lichtquellen 45',45" bzw. deren optischer Eingang und/oder Ausgang sind vorzugsweise über Lichtleiter 48, wie optische Fasern oder optische Faserbündel, gekoppelt.

Der in der Fig. 6 abgebildete Messraum 43 ist lichtdicht ausgebildet und in seiner Grösse bevorzugt so minimiert, dass zumindest eine Mikroplatte 3 und ein diese umgebender Inkubationsrahmen 4 darin so bewegt werden können, dass alle biologische Strukturen enthaltenden Wells 2 der Mikroplatte 3 gegenüber den Aktionsquellen 45',45" und Messeinrichtungen 46,47 des Mikroplatten-Readers 23 bzw. gegenüber den optischen Achsen 51 des Mikroplatten-Readers 23 positioniert werden können. Das minimierte Volumen des Messraums 43 erleichtert das Aufrechterhalten einer feuchten Gasatmosphäre 32 in seinem Innern durch das Verdunsten der Flüssigkeit aus dem Inkubationsrahmen 4, der hier ohne Deckel 12 verwendet wird. Vorzugsweise weist dieser Messraum 43 eine praktisch gasdichte Umhüllung auf, die Wände 52, Boden 53, Decke 54 und Klappe 44 umfasst, und die zum Aufrechterhalten der feuchten Gasatmosphäre 32 ausgebildet ist.

Der erfindungsgemässe Mikroplatten-Reader 23 umfasst zudem vorzugsweise ein Gehäuse 25 und einen internen bzw. integrierten Prozessor 49 oder er ist an einen externen Prozessor (nicht gezeigt) anschliessbar ausgebildet. Ein derartiger Prozessor 49 kann somit ein in die elektronische Steuerung des Mikroplatten-Readers 23 integrierter Mikroprozessor oder ein beigestellter Personal Computer sein.

Gleiche Bezugszeichen in den Figuren bezeichnen gleiche oder zumindest ähnliche Merkmale, auch wenn diese nicht in jedem Fall ausführlich beschrieben sind.

**Bezugszeichen**

| | | | |
|---|---|---|---|
| 1 | Inkubationskassette | 26 | Auflagefläche |
| 2 | Well | 27 | Leseöffnung |
| 3 | Mikroplatte, Standard-Mikroplatte | 28 | Haltestege |
| | | 29 | Halteöffnung |
| 4 | Rahmen, Inkubationsrahmen | 30 | Federriegel |
| 5 | zentrale erste Öffnung, erste Öffnung | 31 | schiefe Anschlagfläche |
| | | 32 | Gasatmosphäre |
| 6 | innere Wand | 33 | Kanalabschnitte |
| 7 | Tragfläche von 4 | 34 | Randteile von 12 |
| 8 | zentrale zweite Öffnung, zweite Öffnung | 35 | Magnetvorrichtung |
| | | 40 | Zentriernocken |
| 9 | äussere Wand | 41 | Absatz |
| 10 | Zwischenboden | 42 | hochragender Teil von 9 |
| 11 | Kanal, Reservoir | 43 | Messraum |
| 12 | Deckel | 44 | Klappe |
| 13 | Platte von 12 | 45' | erste Aktionsquelle |
| 14 | umlaufender Rand von 12 | 45" | zweite Aktionsquelle |
| 15 | umlaufende Spalte | 46 | erste Messeinrichtung |
| 16 | abgesenkter Bereich | 47 | zweite Messeinrichtung |
| 17 | Steg in 11 | 48 | Lichtleiter |
| 18 | Ausschnitt | 49 | Prozessor |
| 19 | Finger | 50 | Steuerung |
| 20 | Ecke von 4 | 51 | optische Achse |
| 21 | Einschnitt | 52 | Messraumwand |
| 22 | Transportauflage | 53 | Messraumboden |
| 23 | Mikroplatten-Reader | 54 | Messraumdecke |
| 24 | magnetisierbare Oberfläche | | |
| 25 | Gehäuse | | |

## Patentansprüche

1. Mikroplatten-Reader (23) zumindest umfassend:
a) einen Messraum (43);
b) eine Aktionsquelle (45'), die zum Herbeiführen einer Wechselwirkung mit biologischen Strukturen in Wells (2) einer Mikroplatte (3) und zum Hervorrufen oder Erzeugen eines messbaren Signals ausgebildet ist;
c) eine Messeinrichtung (46), die zum Erfassen eines Signals ausgebildet ist, das durch biologische Strukturen in Wells (2) einer Mikroplatte (3) ausgesendet oder das durch die Aktionsquelle (45') in oder an biologischen Strukturen in Wells (2) einer Mikroplatte (3) hervorgerufen oder erzeugt wurde; wobei die Messeinrichtung (46) eine optische Achse (51) definiert;
d) eine zumindest teilweise aus dem Messraum (23) ausfahrbare Transportauflage (22), die zum Positionieren von Wells (2) einer Mikroplatte (3) gegenüber der optischen Achse (51) der Messeinrichtung (46) des Mikroplatten-Readers (23) ausgebildet ist, indem die Transportauflage (22) innerhalb des Messraums (43) in zumindest einer Richtung bewegbar ausgebildet ist; und
e) eine Steuerung (50), die zum Steuern der Aktionsquelle (45'), der Messeinrichtung (46) sowie der Bewegungen der Transportauflage (22) des Mikroplatten-Readers (23) ausgebildet ist;
**dadurch gekennzeichnet, dass** der Mikroplatten-Reader (23) eine Inkubationsvorrichtung zum Reduzieren einer Flüssigkeits-Verdunstung aus Wells (2) einer Mikroplatte (3) umfasst, wobei:
(i) die Inkubationsvorrichtung einen Rahmen (4) zum Aufnehmen einer Mikroplatte (3) mit Wellböden aufweisenden Wells (2) umfasst;
(ii) der Rahmen (4) eine von einer inneren Wand (6) umgebene, erste Öffnung (5) umfasst, deren Masse zum Einlegen einer Mikroplatte (3) ausgelegt sind; und
(iii) der Rahmen (4) eine im Wesentlichen parallel zur inneren Wand (6) verlaufende äussere Wand (9) umfasst, die über einen Zwischenboden (10) an der inneren Wand (6) anschliesst, so dass durch die beiden Wände (6,9) und den Zwischenboden (10) ein die erste Öffnung (5) umgebender Kanal (11) zum Aufnehmen einer dem Inhalt der Mikroplattenwells (2) angepassten Flüssigkeit gebildet ist;
**dass** die Inkubationsvorrichtung eine Tragfläche (7) mit einer zweiten Öffnung (8) umfasst, wobei diese Tragfläche (7) an der inneren Wand (6) und zum Tragen einer eingelegten Mikroplatte (3) ausgebildet ist, und wobei infolge der zweiten Öffnung (8) alle Böden der Wells (2) einer in die Inkubationsvorrichtung eingelegten Mikroplatte (3) durch die zweite Öffnung (8) frei zugänglich sind;
**dass** die Transportauflage (22) des Mikroplatten-Readers (23) zumindest eine zum Auflegen oder Abheben der Inkubationsvorrichtung ausgebildete Auflagefläche (26) mit einer Leseöffnung (27) umfasst;
**und dass** die Leseöffnung (27) der Transportauflage (22) in ihrer Grösse und Position der zweiten Öffnung (8) des Rahmens (4) der Inkubationsvorrichtung so angepasst ist, dass alle Böden der Wells (2) einer in die Inkubationsvorrichtung eingelegten Mikroplatte (3) durch die Leseöffnung (27) frei zugänglich sind.

2. Mikroplatten-Reader (23) nach Anspruch 1, **dadurch gekennzeichnet, dass** die Auflagefläche (26) der Transportauflage (22) an den Ecken der Leseöffnung (27) angeordnete Haltestege (28) umfasst, die zum Eingreifen in entsprechende Halteöffnungen (29) in der inneren Wand (6) des Rahmens (4) der Inkubationsvorrichtung ausgebildet sind.

3. Mikroplatten-Reader (23) nach einem der Ansprüche 1 oder 2, **dadurch gekennzeichnet, dass** die Transportauflage (22) einen Federriegel (30) zum Positionieren und Halten einer in den Rahmen (4) der Inkubationsvorrichtung eingelegten Mikroplatte (3) umfasst.

4. Mikroplatten-Reader (23) nach Anspruch 3, **dadurch gekennzeichnet, dass** der Federriegel (30) eine schiefe Anschlagfläche (31) umfasst, die zum Beaufschlagen einer Ecke einer in den Rahmen (4) der Inkubationsvorrichtung eingelegten Mikroplatte (3) ausgebildet ist, wobei dieses Beaufschlagen bewirkt, dass die in die Inkubationsvorrichtung eingelegte Mikroplatte (3) gegen die der schiefen Anschlagfläche (31) gegenüber liegenden Haltestege (28) der Transportauflage (22) gedrückt und exakt auf der Transportauflage (22) positioniert wird.

5. Mikroplatten-Reader (23) nach Anspruch 4, **dadurch gekennzeichnet, dass** der Mikroplatten-Reader (23) ein Gehäuse (25) mit einer integrierten Magnetvorrichtung (35) umfasst, die zum Abheben und Auflegen des Deckels (12) einer auf der Transportauflage (22) aufgelegten Inkubationsvorrichtung ausgebildet ist, wobei die Inkubationsvorrichtung als Inkubationskassette ausgebildet ist.

6. Mikroplatten-Reader (23) nach Anspruch 4, **dadurch gekennzeichnet, dass** der Messraum (43) ein minimiertes Volumen aufweist und eine praktisch gasdichte Umhüllung (44,52,53,54) umfasst, die zum Aufrechterhalten einer feuchten Gasatmosphäre (32) ausgebildet ist, welche durch das Verdunsten der Flüssigkeit aus dem Inkubationsrahmen (4) bewirkt wird.

7. Verwendung eines Mikroplatten-Readers (23) gemäss einem der Ansprüche 1 bis 6 zum Reduzieren der Flüssigkeits-Verdunstung aus Wells (2) von Mikroplatten (3), wobei die Inkubationsvorrichtung als Inkubationskassette (1) mit einem Rahmen (4) und einem Deckel (12) oder als deckelloser Inkubationsrahmen (4) ausgebildet ist.

8. Verwendung nach Anspruch 7, **dadurch gekennzeichnet, dass** der Kanal (11) des Rahmens (4) einer Inkubationsvorrichtung zumindest teilweise mit einer Flüssigkeit gefüllt wird, wobei zum Einfüllen der Flüssigkeit in den Kanal (11) eine Hand-Pipette, eine Pipette einer Labor-Arbeitsstation oder ein Injektor des Mikroplatten-Reader (23) verwendet wird.

9. Verwendung nach Anspruch 8, **dadurch gekennzeichnet, dass** die Inkubationsvorrichtung als Inkubationskassette (1) mit einem Rahmen (4) und einem Deckel (12) ausgebildet ist, **und dass** vor dem Einfüllen der Flüssigkeit in den Kanal (11) einer auf der Transportauflage (22) aufgelegten Inkubationskassette (1) der Deckel (12) der Inkubationskassette (1) mit einer in das Gehäuse (25) des Mikroplatten-Readers (23) integrierten Magnetvorrichtung (35), manuell oder mit einem Roboter einer Labor-Arbeitsstation vom Rahmen (4) der Inkubationskassette (1) abgehoben wird.

10. Verwendung nach Anspruch 9, **dadurch gekennzeichnet, dass** nach dem Einfüllen der Flüssigkeit der Deckel (12) der Inkubationskassette (1) mit einer in das Gehäuse (25) des Mikroplatten-Readers (23) integrierten Magnetvorrichtung (35), manuell oder mit einem Roboter einer Labor-Arbeitsstation auf den Rahmen (4) der Inkubationskassette (1) gelegt wird.

## Claims

1. A microplate reader (23), at least comprising:
a) a measurement chamber (43);
b) an action source (45'), which is designed to produce an interaction with biological structures in wells (2) of a microplate (3) and to induce or generate a measurable signal;
c) a measuring device (46), which is designed to detect a signal, which was emitted by biological structures in wells (2) of a microplate (3) or which was induced or generated by the action source (45') in or on biological structures in wells (2) of a microplate (3); wherein the measuring device (46) defines an optical axis (51);
d) a transportation support (22), which is at least partially extendable from the measurement chamber (23), and which is designed for positioning wells (2) of a microplate (3) in relation to the optical axis (51) of the measuring device (46) of the microplate reader (23), by the transportation support (22) being designed as movable in at least one direction inside the measurement chamber (43); and
e) a controller (50), which is designed for controlling the action source (45'), the measuring device (46), and the movements of the transportation support (22) of the microplate reader (23);
**characterized in that** the microplate reader (23) comprises an incubation device for reducing a liquid evaporation from wells (2) of a microplate (3), wherein:
(i) the incubation device comprises a frame (4) for accommodating a microplate (3) comprising wells (2) having well bottoms;
(ii) the frame (4) comprises a first opening (5), which is enclosed by an inner wall (6), and the dimensions of which are designed for inserting a microplate (3); and
(iii) the frame (4) comprises an outer wall (9) which extends substantially parallel to the inner wall (6), and which adjoins the inner wall (6) via an intermediate bottom (10), so that a channel (11) enclosing the first opening (5) for accommodating a liquid adapted to the content of the microplate wells (2) is formed by the two walls (6, 9) and the intermediate bottom (10);
**that** the incubation device comprises a bearing surface (7) comprising a second opening (8), wherein this bearing surface (7) is formed on the inner wall (6) and is formed to bear an inserted microplate (3), and wherein as a consequence of the second opening (8), all bottoms of the wells (2) of a microplate (3) inserted into the incubation device are freely accessible through the second opening (8);
**that** the transportation support (22) of the microplate reader (23) comprises at least one supporting surface (26), which is designed for depositing or raising the incubation device, comprising a read opening (27);
**and that** the read opening (27) of the transportation support (22) is adapted in its size and position to the second opening (8) of the frame (4) of the incubation device in such a way that all bottoms of the wells (2) of a microplate (3) inserted into the incubation device are freely accessible through the read opening (27).

2. The microplate reader (23) according to Claim 1, **characterized in that** the supporting surface (26) of the transportation support (22) comprises retention webs (28) arranged at the corners of the read opening (27), which are designed to engage in corresponding retention openings (29) in the inner wall (6) of the frame (4) of the incubation device.

3. The microplate reader (23) according to any one of Claims 1 or 2, **characterized in that** the transportation support (22) comprises a spring bolt (30) for positioning and retaining a microplate (3) inserted into the frame (4) of the incubation device.

4. The microplate reader (23) according to Claim 3, **characterized in that** the spring bolt (30) comprises an oblique stop face (31), which is designed to be applied to a corner of a microplate (3) inserted into the frame (4) of the incubation device, wherein this application causes the microplate (3) inserted into the incubation device to be pressed against the retention webs (28) of the transportation support (22) opposite to the oblique stop face (31) and to be positioned exactly on the transportation support (22).

5. The microplate reader (23) according to Claim 4, **characterized in that** the microplate reader (23) comprises a housing (25) having an integrated magnet device (35), which is designed for raising and depositing the cover (12) of an incubation device deposited on the transportation support (22), wherein the incubation device is designed as an incubation cassette.

6. The microplate reader (23) according to Claim 4, **characterized in that** the measurement chamber (43) has a minimized volume and comprises a practically gas-tight casing (44, 52, 53, 54), which is designed to maintain a humid gas atmosphere (32), which is caused by the evaporation of the liquid from the incubation frame (4).

7. A use of a microplate reader (23) according to any one of Claims 1 to 6 for reducing the liquid evaporation from wells (2) of microplates (3), wherein the incubation device is designed as an incubation cassette (1) comprising a frame (4) and a cover (12) or as a cover-free incubation frame (4).

8. The use according to Claim 7, **characterized in that** the channel (11) of the frame (4) of an incubation device is at least partially filled with a liquid, wherein a handheld pipette, a pipette of a laboratory workstation, or an injector of the microplate reader (23) is used for decanting the liquid into the channel (11).

9. The use according to Claim 8, **characterized in that** the incubation device is designed as an incubation cassette (1) comprising a frame (4) and a cover (12), and before the decanting of the liquid into the channel (11) of an incubation cassette (1) deposited on the transportation support (22), the cover (12) of the incubation cassette (1) is raised from the frame (4) of the incubation cassette (1) using a magnet device (35) integrated into the housing (25) of the microplate reader (23), manually, or using a robot of a laboratory workstation.

10. The use according to Claim 9, **characterized in that** after the decanting of the liquid, the cover (12) of the incubation cassette (1) is placed on the frame (4) of the incubation cassette (1) using a magnet device (35) integrated into the housing (25) of the microplate reader (23), manually, or using a robot of a laboratory workstation.

## Revendications

1. Lecteur de microplaques (23) comprenant au moins :
a) une chambre de mesure (43) ;
b) une source d'action (45') conçue pour effectuer une interaction avec des structures biologiques dans les puits (2) d'une microplaque (3) et pour provoquer ou générer un signal mesurable ;
c) un dispositif de mesure (46) conçu pour détecter un signal qui est émis par des structures biologiques dans les puits (2) d'une microplaque (3) ou qui a été provoqué ou généré par la source d'action (45') dans ou sur des structures biologiques dans les puits (2) d'une microplaque (3), le dispositif de mesure (46) définissant un axe optique (51) ;
d) un support de transport (22) pouvant être déployé au moins partiellement de la chambre de mesure (23), conçu pour positionner les puits (2) d'une microplaque (3) par rapport à l'axe optique (51) du dispositif de mesure (46) du lecteur de microplaques (23), le support de transport (22) étant réalisé mobile à l'intérieur de la chambre de mesure (43) dans au moins une direction ; et
e) une commande (50) conçue pour commander la source d'action (45'), le dispositif de mesure (46) et les déplacements du support de transport (22) du lecteur de microplaques (23) ;
**caractérisé en ce que** le lecteur de microplaques (23) comprend un dispositif d'incubation pour réduire l'évaporation de liquide à partir des puits (2) d'une microplaque (3), dans lequel :
(i) le dispositif d'incubation comprend un cadre (4) destiné à recevoir une microplaque (3) avec des puits (2) présentant des fonds de puits ;
(ii) le cadre (4) comprend un premier orifice (5) entouré d'une paroi intérieure (6), dont les dimensions sont conçues pour introduire une microplaque (3) ; et
(iii) le cadre (4) comprend une paroi extérieure (9) s'étendant essentiellement parallèlement à la paroi intérieure (6) qui se raccorde à la paroi intérieure (6) par un fond intermédiaire (10) de telle façon que les deux parois (6, 9) et le fond intermédiaire (10) forment un canal (11) entourant le premier orifice (5) et destiné à recevoir un liquide adapté au contenu des puits (2) de la microplaque ;
**en ce que** le dispositif d'incubation comprend une surface d'appui (7) avec un deuxième orifice (8), cette surface d'appui (7) étant conçue sur la paroi intérieure (6) pour porter une microplaque (3), et le deuxième orifice (8) permettant d'accéder librement à tous les fonds du puits (2) d'une microplaque (3) introduite dans le dispositif d'incubation ;
**en ce que** le support de transport (22) du lecteur de microplaques (23) comprend au moins une surface de support (26) avec une ouverture de lecture (27) pour poser ou soulever le dispositif d'incubation ;
**et en ce que** l'ouverture de lecture (27) du support de transport (22) est adaptée de par ses dimensions et la position du deuxième orifice (8) du cadre (4) du dispositif d'incubation pour accéder librement par l'ouverture de lecture (27) à tous les fonds des puits (2) d'une microplaque (3) introduite dans le dispositif d'incubation.

2. Lecteur de microplaques (23) selon la revendication 1, **caractérisé en ce que** la surface de support (26) du support de transport (22) comprend des barrettes de retenue (28) agencées aux coins de l'ouverture de lecture (27) conçues pour venir s'engager dans des ouvertures de retenues correspondantes (29) dans la paroi intérieure (6) du cadre (4) du dispositif d'incubation.

3. Lecteur de microplaques (23) selon l'une des revendications 1 ou 2, **caractérisé en ce que** le support de transport (22) comprend un verrou à ressort (30) pour positionner et maintenir une microplaque (3) introduite dans le cadre (4) du dispositif d'incubation.

4. Lecteur de microplaques (23) selon la revendication 3, **caractérisé en ce que** le verrou à ressort (30) comprend une surface de butée oblique (31) conçue pour venir s'appliquer au niveau d'un angle d'une microplaque (3) introduite dans le cadre (4) du dispositif d'incubation, cette application faisant que la microplaque (3) introduite dans le dispositif d'incubation est appuyée contre la barrette de retenue (28) du support de transport (22) agencée à l'opposé de la surface de butée oblique (31) et est positionnée de façon précise sur le support de transport (22).

5. Lecteur de microplaques (23) selon la revendication 4, **caractérisé en ce que** le lecteur de microplaques (23) comprend un boîtier (25) avec un dispositif magnétique intégré (35) conçu pour retirer et poser le couvercle (12) d'un dispositif d'incubation posé sur le support de transport (22), le dispositif d'incubation étant réalisé sous la forme d'une cassette d'incubation.

6. Lecteur de microplaques (23) selon la revendication 4, **caractérisé en ce que** la chambre de mesure (43) présente un volume minimal et comprends une enveloppe pratiquement étanche au gaz (44, 52, 53, 54) conçue pour maintenir une atmosphère gazeuse humide obtenue par évaporation du liquide à partir du cadre d'incubation (4).

7. Utilisation d'un lecteur de microplaques (23) selon l'une des revendications 1 à 6 pour réduire l'évaporation du liquide à partir des puits (2) des microplaques (3), le dispositif d'incubation étant réalisé sous la forme d'une cassette d'incubation (1) avec un cadre (4) et un couvercle (12) ou sous la forme d'un cadre d'incubation (4) sans couvercle.

8. Utilisation selon la revendication 7, **caractérisée en ce que** le canal (11) du cadre (4) d'un dispositif d'incubation est rempli au moins en partie d'un liquide, dans laquelle, pour remplir le liquide dans le canal (11) on utilise une pipette à main, une pipette d'un poste de travail en laboratoire ou un injecteur du lecteur de microplaques (23).

9. Utilisation selon la revendication 8, **caractérisée en ce que** le dispositif d'incubation est réalisé sous la forme d'une cassette d'incubation (1) avec un cadre (4) et un couvercle (12), et **en ce que** avant de remplir le liquide dans le canal (11) d'une cassette d'incubation (1) posée sur le support de transport (22), le couvercle (12) de la cassette d'incubation (1) est retiré du cadre (4) de la cassette d'incubation (1) avec un dispositif magnétique (35) intégré dans le boîtier (25) du lecteur de microplaques (23), à la main, ou avec un robot d'un poste de travail en laboratoire.

10. Utilisation selon la revendication 9, **caractérisée en ce que,** après le remplissage, le couvercle (12) de la cassette d'incubation (1) est posé sur le cadre (4) de la cassette d'incubation (1) avec un dispositif magnétique (35) intégré dans le boîtier (25) du lecteur de microplaques (23), à la main, ou avec un robot d'un poste de travail en laboratoire.
